# EUROPEAN PATENT APPLICATION

(11) **EP 4 299 051 A1**
(43) Date of publication of application: **03.01.2024**
(21) Application number: 23181679.4
(22) Date of filing: 27.06.2023
(51) Int. Cl.: A61K 8/9794, A61K 8/9789, A61K 8/49, A61Q 19/08, A61Q 19/00, A61K 31/352, A61K 36/82, A61K 36/8962, A61K 36/61, A61P 17/10, A61P 21/00

(54) **COSMETIC COMPOSITION INCLUDING GARLIC EXTRACT, BLACK TEA EXTRACT, AND GUAVA EXTRACT**

(30) Priority: 28.06.2022 KR 20220079283; 16.03.2023 KR 20230034573
(71) Applicant: Cosmax, Inc., Hwaseong-si, Gyeonggi-do 18622 (KR)
(72) Inventor: HEO, Young Mok, SEONGNAM-SI (KR); LEE, Dong Geol, 18496 HWASEONG-SI (KR); KANG, Seung Hyun, 06285 SEOUL (KR); PARK, Myeong Sam, SEONGNAM-SI (KR); KIM, Hye Youn, SEONGNAM-SI (KR); NHO, Youn Hwa, SEONGNAM-SI (KR)
(74) Representative: Lavoix

(57) **Abstract**

Provided is a cosmetic composition which can ameliorate wrinkles and suppress secretion of sweat and sebum, thereby exhibiting a makeup lasting effect.

## Description

### CROSS-REFERENCE TO RELATED APPLICATIONS

This application is based on and claims priority under 35 U.S.C. §119 to Korean Patent Application Nos. 10-2022-0079283, filed on June 28, 2022, and 10-2023-0034573, filed on March 16, 2023 in the Korean Intellectual Property Office, the disclosures of which are incorporated by reference herein in its entirety.

### BACKGROUND

### 1. Field

The disclosure relates to a cosmetic composition including an extract of a natural product.

### 2. Description of the Related Art

Color cosmetics were used by ancient people for the purposes of beautification, body protection, and tribe preservation. However, in modern times, color cosmetics have been away from the traditional purpose and are used to express skin color uniformly and beautifully through coloring of the skin. By using the effect of colors, the face can have harmony and aesthetic functions, and at the same time, can be seen strong and attractive.

However, these color cosmetics are affected by secretions secreted from the human body, such as sebum, sweat, tears around the eyes, and saliva around the mouth, which are continuously secreted through hair follicles and sweat glands. Also, due to the influence of physical contact that occurs during daily activities, the makeup collapses and fails to maintain the function at the beginning of application.

Therefore, in order to solve these problems, through the development of various raw materials and formulations, there is a need to develop products with excellent durability that increase skin adhesion and do not smudge makeup.

### SUMMARY

Provided is a composition for improving a skin condition, including, as an active ingredient, a garlic extract, a black tea extract, and a guava extract.

Provided is a composition for improving a skin condition, including myricetin as an active ingredient.

Provided is a method of preventing, improving, or treating a condition of a subject, the method including treating or administering an effective amount of the composition to a subject in need thereof.

Additional aspects will be set forth in part in the description which follows and, in part, will be apparent from the description, or may be learned by practice of the presented embodiments of the disclosure.

According to an aspect of the disclosure, a composition for improving a skin condition includes, as an active ingredient, a garlic extract, a black tea extract, and a guava extract.

The term "extract" as used herein refers to all substances obtained by extracting components of natural substances, and also refers to substances obtainable by extracting components of natural substances and then processing or treating the extracted products by other methods. For example, the processing or treatment may include dilution, concentration, drying, purification, fractionation, filtration, fermentation, enzymatic treatment, and the like. The processing or treatment may be carried out by methods in the art. Therefore, the extraction product may include an extract, a dilution or concentrate of the extract, a dried product obtained by drying the extract, or a crude or purified product of the aforementioned product, or a fraction obtained by fractionating the aforementioned product.

The garlic extract, black tea extract, or guava extract may be obtained by extracting garlic, black tea, or guava with water, C1-C4 lower alcohol, or a mixture thereof as a solvent. The lower alcohol may include, for example, methanol, ethanol, glycerin, propylene glycol, butylene glycol, or the like. The lower alcohol may be ethanol. The solvent may be 50 %(v/v) to 99 %(v/v) ethanol, 50 %(v/v) to 80 %(v/v) ethanol, or 70 %(v/v) ethanol. In detail, after cutting garlic, black tea, or guava, extraction may be performed thereon by adding water, alcohol, or a mixture thereof 2 to 20 times the weight of the garlic, black tea, or guava. In addition, the extraction process may be performed at a temperature in a range of about 30 °C to about 100 °C for about 1 hour to about 120 hours. For the extraction, a cold extraction method, an ultrasonic extraction method, a reflux extraction method, or the like may be used, and the number of extraction may be performed for 1 time to 5 times. In addition, leaves, stems, flowers, roots, peels, fruits, or mixtures thereof of the garlic, black tea or guava may be targets to be extracted.

The garlic extract, black tea extract, and guava extract may be mixed at a weight ratio in a range of about 1:0.1:0.1 to about 1:10:10. The garlic extract, black tea extract, and guava extract may be, for example, mixed at a weight ratio in a range of about 1:0.1:0.1 to about 1:10:10, about 1:0.1:0.1 to about 1:5:5, about 1:0.1:0.1 to about 1:2:2, about 1:0.5:0.5 to about 1:5:5, about 1:0.5:0.5 to about 1:2:2, about 1:0.5:0.5 to about 1:1.5:1.5, about 1:0.1:0.5 to about 1:8:10, about 1:0.1:1 to about 1:5:10, about 1:5:5 to about 1:10:10, about 1:5:5 to about 1:8:8, or about 1:7:7 to about 1:9:9. The weight ratio of the garlic extract, black tea extract, and guava extract may be 1 to 100:1 to 100:1 to100, 1 to 10:1 to 10:1 to 10, 1 to 5:1 to 5:1 to 5, or 1 to 2:1 to 2:1 to 2, and for example, 1:1:1. Here, when the mixing ratio of the garlic extract, black tea extract, and guava extract is less than or exceeds the ranges above, the synergistic effect of improving a skin condition by the garlic extract, black tea extract, and guava extract is not sufficiently exhibited.

The garlic extract, black tea extract, or guava extract may be included in an amount in a range of about 0.001 wt% to about 50 wt%, respectively, based on the total weight of the composition. The amount of the garlic extract, black tea extract, or guava extract may be, based on the total weight of the composition, for example, in a range of about 0.001 wt% to about 50 wt%, about 0.001 wt% to about 40 wt%, about 0.001 wt% to about 30 wt%, about 0.001 wt% to about 20 wt%, about 0.001 wt% to about 10 wt%, about 0.01 wt% to about 50 wt%, about 0.01 wt% to about 40 wt%, about 0.01 wt% to about 30 wt%, about 0.01 wt% to about 20 wt%, about 0.01 wt% to about 10 wt%, about 0.01 wt% to about 5 wt%, about 0.05 wt% to about 50 wt%, about 0.05 wt% to about 40 wt%, about 0.05 wt% to about 30 wt%, about 0.05 wt% to about 20 wt%, about 0.05 wt% to about 10 wt%, about 0.05 wt% to about 5 wt%, about 0.1 wt% to about 50 wt%, about 0.1 wt% to about 40 wt%, about 0.1 wt% to about 30 wt%, about 0.1 wt% to about 20 wt%, about 0.1 wt% to about 10 wt%, or about 0.1 wt% to about 5 wt%, respectively. Here, when the amount of the garlic extract, black tea extract, or guava extract is less than or exceeds the ranges above, the synergistic effect of improving a skin condition by the garlic extract, black tea extract, and guava extract is not sufficiently exhibited.

The composition may include, based on the total weight of the composition, a mixture of the garlic extract, the black tea extract, and the guava extract in an amount in a range of about 0.001 wt% to about 80 wt%, for example, about 0.01 wt% to about 60 wt%, about 0.01 wt% to about 40 wt%, about 0.01 wt% to about 30 wt%, about 0.01 wt% to about 20 wt%, about 0.01 wt% to about 10 wt%, about 0.01 wt% to about 5 wt%, about 0.05 wt% to about 60 wt%, about 0.05 wt% to about 40 wt%, about 0.05 wt% to about 30 wt%, about 0.05 wt% to about 20 wt%, about 0.05 wt% to about 10 wt%, about 0.05 wt% to about 5 wt%, about 0.1 wt% to about 60 wt%, about 0.1 wt% to about 40 wt%, about 0.1 wt% to about 30 wt%, about 0.1 wt% to about 20 wt%, about 0.1 wt% to about 10 wt%, or about 0.1 wt% to about 5 wt%.

The composition including the mixture of the garlic extract, the black tea extract, and the guava extract may include myricetin. The myricetin may be an active ingredient of the mixture.

In the Example, it is confirmed that the composition including the garlic extract, the black tea extract, and the guava extract inhibits the formation of SNARE complex, and thus the membrane fusion rate is significantly reduced. It is also confirmed that, in skin tissue, the composition significantly reduces the level of VAMP2, which is one of SNARE proteins, and the release of a neurotransmitter, acetylcholine. Accordingly, it is confirmed that, by acting specifically on the SNARE proteins, the composition inhibits the formation of SNARE complex so that the membrane fusion is accordingly inhibited, and accordingly, the release of neurotransmitters is inhibited. These effects may be exhibited due to the myricetin which is an active ingredient of the extract.

That is, since the composition according to an aspect inhibits the formation of SNARE complex involved in neurotransmission pathways by acting on neuromuscular junctions, an effect of paralyzing motor nerve terminals is exhibited, and thus the repetitive contraction and relaxation of skin muscles can be inhibited. Also, the composition not only inhibits the secretion of sweat and/or sebum by blocking the release of neurotransmitters and inhibits the generation of wrinkles by musculation or sympathetic and parasympathetic nerves, but also ameliorates wrinkles already generated.

Therefore, the composition according to an aspect may have an effect of improving a skin condition. The skin condition may include, for example, amelioration of skin wrinkles or elasticity, reduction in the appearance of fine lines and wrinkles, widening of the eyes, lifting of the corner of the mouth, reduction in muscle mass, flattening of the line extending from the upper lip, and the like. Thus, the composition may replace botox, which is used for the purpose of ameliorating wrinkles through muscle paralysis, by including extracts of non-toxic natural plants.

Also, the composition may be used for suppressing sebum secretion or improving or enhancing makeup durability.

Also, the composition may be used for preventing or treating neuromuscular-related diseases. The neuromuscular-related disease may be, for example, muscle tension, muscle spasm, hemifacial spasm, adult onset spasmodic torticollis, anal fissure, blepharospasm, facial muscle spasm, cerebral palsy, headache, migraine, muscle pain, strabismus, temporomandibular joint disorder, nerve pain, overactive bladder, urgency urinary incontinence, rhinitis, paranasal sinusitis, acne, pore enlargement, dystonia, dystonic contraction, hyperhidrosis, vocal cord dysfunction, myocardial damage, or the like.

Also, in one or more embodiments, the composition may further include a guava leaf extract, a luffa cylindrica extract, and a water lily flower extract. An extraction solvent, an extraction method, an extraction target, etc. for the guava leaf extract, the luffa cylindrica extract, and the water lily flower extract are the same as described herein.

The guava leaf extract, the luffa cylindrica extract, or the water lily flower extract may be included in an amount in a range of about 0.001 wt% to about 50 wt%, respectively, based on the total weight of the composition. The amount of the guava leaf extract, the luffa cylindrica extract, or the water lily flower extract may be, based on the total weight of the composition, for example, in a range of about 0.001 wt% to about 50 wt%, about 0.001 wt% to about 40 wt%, about 0.001 wt% to about 30 wt%, about 0.001 wt% to about 20 wt%, about 0.001 wt% to about 10 wt%, about 0.01 wt% to about 50 wt%, about 0.01 wt% to about 40 wt%, about 0.01 wt% to about 30 wt%, about 0.01 wt% to about 20 wt%, about 0.01 wt% to about 10 wt%, about 0.01 wt% to about 5 wt%, about 0.05 wt% to about 50 wt%, about 0.05 wt% to about 40 wt%, about 0.05 wt% to about 30 wt%, about 0.05 wt% to about 20 wt%, about 0.05 wt% to about 10 wt%, about 0.05 wt% to about 5 wt%, about 0.1 wt% to about 50 wt%, about 0.1 wt% to about 40 wt%, about 0.1 wt% to about 30 wt%, about 0.1 wt% to about 20 wt%, about 0.1 wt% to about 10 wt%, or about 0.1 wt% to about 5 wt%, respectively. Here, when the amount of the guava leaf extract, the luffa cylindrica extract, or the water lily flower extract is less than or exceeds the ranges above, there is a problem in that the effect of improving a skin condition is not sufficiently exhibited.

Also, the garlic extract, the black tea extract, the guava extract, the guava leaf extract, the luffa cylindrica extract, and the water lily flower extract may be mixed at a weight ratio in a range of 1 to 0:1 to 10:1 to 10:1 to 10:1 to 10:1 to 10. The garlic extract, the black tea extract, the guava extract, the guava leaf extract, the luffa cylindrica extract, and the water lily flower extract may be, for example, mixed at a weight ratio in a range of 1 to 5:1 to 5:1 to 5:1 to 5:1 to 5:1 to 5, 2 to 4:2 to 4:2 to 4:1 to 3:1 to 3:4 to 10, or 1.5:1.5:1.5:1:1:3. Here, when the mixing ratio of the garlic extract, the black tea extract, the guava extract, the guava leaf extract, the luffa cylindrica extract, and the water lily flower extract is less than or exceed the ranges above, garlic extract, there is a problem that the synergistic effect of skin condition amelioration by the black tea extract, the guava extract, the guava leaf extract, the luffa cylindrica extract, and the water lily flower extract is not sufficiently exhibited.

The composition may include, based on the total weight of the composition, a mixture of the garlic extract, the black tea extract, the guava extract, the guava leaf extract, the luffa cylindrica fruit extract, and the water lily flower extract in an amount in a range of about 0.001 wt% to about 80 wt%, for example, about 0.01 wt% to about 60 wt%, about 0.01 wt% to about 40 wt%, about 0.01 wt% to about 30 wt%, about 0.01 wt% to about 20 wt%, about 0.01 wt% to about 10 wt%, about 0.01 wt% to about 5 wt%, about 0.05 wt% to about 60 wt%, about 0.05 wt% to about 40 wt%, about 0.05 wt% to about 30 wt%, about 0.05 wt% to about 20 wt%, about 0.05 wt% to about 10 wt%, about 0.05 wt% to about 5 wt%, about 0.1 wt% to about 60 wt%, about 0.1 wt% to about 40 wt%, about 0.1 wt% to about 30 wt%, about 0.1 wt% to about 20 wt%, about 0.1 wt% to about 10 wt%, or about 0.1 wt% to about 5 wt%.

The composition including the mixture of the garlic extract, the black tea extract, the guava extract, the guava leaf extract, the luffa cylindrica fruit extract, and the water lily flower extract may include myricetin. The myricetin may be an active ingredient of the mixture.

In the Example, the composition including the garlic extract, the black tea extract, and the guava extract, specifically, the composition including the garlic extract, the black tea extract, the guava extract, the guava leaf extract, the luffa cylindrica fruit extract, and the water lily flower extract is confirmed to suppress or decrease the sebum secretion and enhance the makeup durability. Therefore, the composition according to an embodiment may be used to suppress the sebum secretion or improve or enhance the makeup durability.

According to another aspect of the disclosure, a composition for improving a skin condition includes myricetin as an active ingredient.

The myricetin may be: derived from chemical synthesis; derived from biosynthesis using microorganisms; or derived from plants or mushrooms. To obtain the myricetin from plants or mushrooms, methods known in the art may be used.

The plants and mushrooms may be selected from broccoli, cauliflower, cabbage, Chinese cabbage, kalian, green chili, red chili, bell pepper, bird chili, Chinese chives, onion, garlic, belimbi, yam, tapioca, sweet potato, papaya, cashew, fern, soybean, mungbean, okra (lady's finger), winged bean, French bean, pea (French pea), string bean, petai, peria (bitter melon), brinjal (eggplant), angular loofa (Chinese okra), snake gourd, pumpkin, sengkuang (jícama), guava, carrot, white radish (daikon), red spinach, bayam duri, kangkung, wolfberry (goji), drumstick (moringa), celery, limau purut, daun turi (vegetable hummingbird), betel, pandan, lemon, semambu (Calamus scipionum), kesom (Polygonum minus), maman (Gynandropsis gynandra), kadok (Piper sarmentosum), cekur manis, selom, pegaga, bunga kantan, plantain, asam gelugor, turmeric, mint, oyster mushroom, and black tea, but embodiments of the present disclosure are not limited thereto.

The myricetin may be included in the composition at a concentration in a range of about 0.1 µm to about 1,000 µm, about 0.1 µm to about 500 µm, about 0.1 µm to about 200 µm, about 1 µm to about 1, 000 µm, about 1 µm to about 500 µm, about 1 µm to about 200 µm, about 10 µm to about 1,000 µm, about 10 µm to about 500 µm, about 10 µm to about 200 µm, about 50 µm to about 1,000 µm, about 50 µm to about 500 µm, about 50 µm to about 200 µm, or about 50 µm to about 150 µm. The myricetin may be included in the composition at a concentration in a range of about 0.001 µg/mL to about 100 µg/mL, about 0.001 µg/mL to about 50 µg/mL, about 0.001 µg/mL to about 20 µg/mL, about 0.001 µg/mL to about 10 µg/mL, about 0.01 µg/mL to about 100 µg/mL, about 0.01 µg/mL to about 50 µg/mL, about 0.01 µg/mL to about 20 µg/mL, about 0.01 µg/mL to about 10 µg/mL, about 0.1 µg/mL to about 100 µg/mL, about 0.1 µg/mL to about 50 µg/mL, about 0.1 µg/mL to about 20 µg/mL, or about 0.1 µg/mL to about 10 µg/mL. When the concentration of the myricetin is less than or exceeds the ranges above, the effect of improving a skin condition may not be sufficiently exhibited.

In the Example, the composition including the garlic extract, the black tea extract, and the guava extract, specifically, the composition including the garlic extract, the black tea extract, the guava extract, the guava leaf extract, the luffa cylindrica fruit extract, and the water lily flower extract is confirmed to include the myricetin as an active ingredient.

In the Example, it is confirmed that the myricetin inhibits the formation of SNARE complex and significantly reduces the membrane fusion rate. It is also confirmed that the myricetin significantly reduces, in skin tissue, the level of VAMP2, which is one of SNARE proteins, and the release of a neurotransmitter, acetylcholine. Accordingly, it is confirmed that, by acting specifically on the SNARE proteins, the myricetin inhibits the formation of SNARE complex so that the membrane fusion is accordingly inhibited, and accordingly, the release of neurotransmitters is inhibited. Accordingly, the composition not only inhibits the secretion of sweat and/or sebum by blocking the release of neurotransmitters and inhibits the generation of wrinkles by musculation or sympathetic and parasympathetic nerves, but also ameliorates wrinkles already generated.

Therefore, the composition according to an aspect may have an effect of improving a skin condition. The skin condition may include, for example, amelioration of skin wrinkles or elasticity, reduction in the appearance of fine lines and wrinkles, widening of the eyes, lifting of the corner of the mouth, reduction in muscle mass, flattening of the line extending from the upper lip, and the like.

Also, the composition may be used for suppressing sebum secretion or improving or enhancing makeup durability.

Also, the composition may be used for preventing or treating neuromuscular-related diseases. The neuromuscular-related disease may be, for example, muscle tension, muscle spasm, hemifacial spasm, adult onset spasmodic torticollis, anal fissure, blepharospasm, facial muscle spasm, cerebral palsy, headache, migraine, muscle pain, strabismus, temporomandibular joint disorder, nerve pain, overactive bladder, urgency urinary incontinence, rhinitis, paranasal sinusitis, acne, pore enlargement, dystonia, dystonic contraction, hyperhidrosis, vocal cord dysfunction, myocardial damage, or the like.

The expression "including an active ingredient" as used herein refers that the extract of the present specification is added to the extent that the aforementioned effect can be exhibited, and that various components are added as subcomponents to be formulated in various forms for drug delivery and stabilization.

In an embodiment, the composition may be in a liquid state or a dry state. In an embodiment, the composition may be in the form of dry powder.

In an embodiment, a drying method for preparing the composition in a dray state may use methods generally used in the art, and is not particularly limited. Non-limiting examples of the drying method are an air drying method, a natural dry method, a spray drying method, a freeze drying method, and the like. These methods may be used alone or at least two methods may be used together.

In an embodiment, the composition may include an effective amount of an additive sufficient to reduce deterioration of the extract. The additive may be, for example, a binding agent, but embodiments are not limited thereto.

In an embodiment, the composition may further include a carrier that is cosmetically acceptable, pharmaceutically acceptable, or acceptable in food. The composition may be formulated with a carrier and provided as a cosmetic, a drug, a food additive, or the like.

In an embodiment, the composition may be a cosmetic composition.

The cosmetic composition may be, for example, a softening lotion, a nutrient lotion, a massage cream, a nutrient cream, an essence, a pack, a gel, an ampoule, or a skin-adhesive cosmetic formulation.

The components included in the cosmetic composition may include components that are commonly used in the cosmetic composition, in addition to the composition as an active ingredient. For example, the components may include common auxiliary agents and carriers, such as a stabilizer, a solubilizer, a vitamin, a pigment, and a flavor.

In an embodiment, the composition may be a composition for external skin application.

The external skin application may be a cream, a gel, an ointment, a skin emulsifier, a skin suspension, a transdermal delivery patch, a drug-containing bandage, a lotion, or a combination thereof. For the external skin application, components used in ordinary cosmetics or external skin applications, such as water-based ingredients, oil-based components, oil-based ingredients, powdered ingredients, alcohols, moisturizers, thickeners, ultraviolet ray absorbents, whitening agents, preservatives, antioxidants, surfactants, flavoring agents, colorants, various skin nutrients, or a combination thereof, may be suitably blended as needed. For the external skin application, a sequestrant, such as disodium edetate, trisodium edetate, trisodium citrate, sodium polyphosphate, sodium metaphosphate, gluconic acid, or the like; a medicine, such as caffeine, tannin, verapamil, a licorice extract, glabridin, a hot water extract of caline fruit, various herbal medicines, tocopherol acetate, glycyrrhizic acid, tranexamic acid, a derivative thereof, or a salt or the like thereof; vitamin C, magnesium ascorbyl phosphate, ascorbyl glucoside, albutin, kojic acid, or sugar, such as glucose, fructose, trehalose, or the like, may be suitably blended.

In an embodiment, the composition may be a pharmaceutical composition.

The pharmaceutical composition may be used for preventing or treating neuromuscular-related diseases.

The pharmaceutical composition may further include a diluent or a carrier. The diluent may include lactose, corn starch, soybean oil, microcrystalline cellulose, or mannitol, and a glidant may include magnesium stearate, talc, or a combination thereof. The carrier may include an excipient, a disintegrant, a binding agent, a glidant, or a combination thereof. The excipient may include microcrystalline cellulose, lactose, low-substituted hydroxy cellulose, or a combination thereof. The disintegrant may include carboxymethyl cellulose calcium, sodium starch glycolate, anhydrous calcium hydrogen phosphate, or a combination thereof. The binding agent may include polyvinyl pyrrolidone, low-substituted hydroxypropyl cellulose, hydroxypropyl cellulose, or a combination thereof. The glidant may include magnesium stearate, silicon dioxide, talc, or a combination thereof.

The pharmaceutical composition may be formulated for oral or parenteral administration. The formulation for oral administration may include granule, powder, liquid, tablet, capsule, dry syrup, or a combination thereof. The formulation for parenteral administration may include injection.

In an embodiment, the composition may be a health functional food composition.

For the health functional food composition, the composition may be used alone or in combination with other foods or food ingredients, and may be used according to methods in the art. A mixing amount of the active ingredient may be appropriately determined depending on the purpose of use (prevention, health or therapeutic treatment). In general, when preparing food or beverage, the composition disclosed herein may be added in an amount of 15 parts by weight or less with respect to the raw materials. Types of the health functional food are not particularly limited. Among the types of the health functional food, a beverage composition may contain, as additive ingredients, various flavoring agents or natural carbohydrates as in general beverages. The native carbohydrate may include monosaccharides, such as glucose and fructose, disaccharides, such as maltose and sucrose, polysaccharides, such as dextrin and cyclodextrin, and sugar alcohols, such as xylitol, sorbitol, and erythritol. For use as a sweetening agent, a natural sweetening agent, such as thaumatin and a stevia extract, and a synthetic sweetening agent, such as saccharin, aspartame, etc., may be used. The health food composition may also contain a nutrient, a vitamin, an electrolyte, a flavoring agent, a colorant, pectic acid and a salt thereof, alginic acid and a salt thereof, organic acid, a protective colloidal thickener, a pH adjuster, a stabilizer, a preservative, glycerin, alcohol, and a carbonizing agent used in carbonated beverages, or a combination thereof. The health functional food composition may also include natural fruit juice and fruit flesh for preparing fruit juice beverage and vegetable beverage, or a combination thereof.

Also, another aspect provides a method of preventing, improving, or treating a condition of a subject, the method including treating or administering an effective amount of the composition to a subject in need thereof.

The condition of the subject may be a skin-related condition or a neuromuscular-related disease. The skin-related condition or the neuromuscular-related disease is the same as described above.

The terms "administering", "introducing", and "grafting" as used in the present specification may be used interchangeably, and may be construed as arranging a composition according to an embodiment in a subject by a method or pathway that causes at least partial localization on a desired site.

The administration may be performed by a method known in the art. The administration may be performed directly to a subject by any means, for example, intravenous, intramuscular, oral, transdermal, mucosal, intranasal, intratracheal, or subcutaneous routes. The administration may be administered systemically or locally.

The subject may be a mammal, for example, a human, a cow, a horse, a pig, a dog, a sheep, a goat, or a cat. The subject may be: a subject in need of effects of improving skin condition, such as skin wrinkles or elasticity, or inhibiting sebum secretion; or a subject in need of prevention or treatment of neuromuscular-related diseases.

For the administration, the composition according to an embodiment may be administered to a subject at a daily dosage in a range of about 0.1 mg to about 1,000 mg, for example, about 0.1 mg to about 500 mg, about 0.1 mg to about 100 mg, about 0.1 mg to about 50 mg, about 0.1 mg to about 25 mg, about 1 mg to about 1,000 mg, about 1 mg to about 500 mg, about 1 mg to about 100 mg, about 1 mg to about 50 mg, about 1 mg to about 25 mg, about 5 mg to about 1,000 mg, about 5 mg to about 500 mg, about 5 mg to about 100 mg, about 5 mg to about 50 mg, about 5 mg to about 25 mg, about 10 mg to about 1,000 mg, about 10 mg to about 500 mg, about 10 mg to about 100 mg, about 10 mg to about 50 mg, or about 10 mg to about 25 mg. However, the dosage may be variously prescribed depending on factors, such as a formulation method, an administration method, age, weight, gender, and medical conditions of a patient, food, administration time, an administration route, an excretion rate, and response sensitivity, and a person skilled in the art may appropriate adjust the dosage in consideration of these factors. The number of administration may be once a day or at least twice a day within the range of clinically acceptable side effects, and the administration may be performed at a single site or at least two sites, daily or every 2 days to 5 days. The total number of administration days may be 1 day to 30 days per treatment. As needed, the same treatment may be repeated after the a suitable period of time. For animals other than humans, the same dosage per kg as for humans may be used, or for example, a dosage converted from the aforementioned dosage by the volume ratio (e.g., average value) of the organ (e.g., heart, etc.) between a target animal and the human may be used.

### BRIEF DESCRIPTION OF THE DRAWINGS

The above and other aspects, features, and advantages of certain embodiments of the disclosure will be more apparent from the following description taken in conjunction with the accompanying drawings, in which:
FIG. 1 shows a result of confirming membrane fusion rate of a composition according to an embodiment;
FIG. 2 shows a result of confirming whether or not myricetin is detected and amount of detected myricetin;
FIG. 3 shows a result of confirming the VAMP2 level by treatment with the composition or myricetin according to an embodiment;
FIG. 4 shows a result of confirming the acetylcholine level by treatment with the composition or myricetin according to an embodiment;
FIG. 5 shows a result of confirming changes in an amount of skin sebum by the composition according to an embodiment for each time period;
FIG. 6 shows a result of confirming changes in skin color by the composition according to an embodiment for each time period; and
FIG. 7 shows a result of confirming rates of skin color change by the composition according to an embodiment for each time period.

### DETAILED DESCRIPTION

Reference will now be made in detail to embodiments, examples of which are illustrated in the accompanying drawings, wherein like reference numerals refer to like elements throughout. In this regard, the present embodiments may have different forms and should not be construed as being limited to the descriptions set forth herein. Accordingly, the embodiments are merely described below, by referring to the figures, to explain aspects of the present description. As used herein, the term "and/or" includes any and all combinations of one or more of the associated listed items. Expressions such as "at least one of," when preceding a list of elements, modify the entire list of elements and do not modify the individual elements of the list.

Hereinafter, preferable Examples are presented to help understanding of the present disclosure. However, the following examples are only presented for easier understanding of the present disclosure, and the contents of the present disclosure are not limited by the following examples.

### [Preparation Examples]

### Preparation Example 1. Preparation of garlic extract

Fresh garlics grown on the farm were harvested and washed thoroughly with distilled water. After drying the garlics for a week to remove moisture, 100 g of the dried garlics was pulverized and immersed in 1,000 g of 70 % ethanol (EtOH), followed by stirring and extraction at room temperature for 3 days. Afterwards, the extract was filtered by centrifugation, and the filtrate was repeatedly extracted twice in the same manner as described above. The extract from which the filtrate was removed was concentrated with a rotary vacuum concentrator to prepare a concentrate, and then freeze-dried to prepare a garlic extract.

### Preparation Example 2. Preparation of black tea extract

A black tea extract was prepared in the same manner as in Preparation Example 1, except that 100 g of black tea was used.

### Preparation Example 3. Preparation of guava extract

A guava extract was prepared in the same manner as in Preparation Example 1, except that 100 g of guava was used.

### Preparation Example 4. Preparation of guava leaf extract

A guava leaf extract was prepared in the same manner as in Preparation Example 1, except that 100 g of guava leaf was used.

### Preparation Example 5. Preparation of luffa cylindrica fruit extract

A luffa cylindrica fruit extract was prepared in the same manner as in Preparation Example 1, except that 100 g of luffa cylindrica fruit was used.

### Preparation Example 6. Preparation of water lily flower extract

A water lily flower extract was prepared in the same manner as in Preparation Example 1, except that 100 g of water lily flower was used.

### [Examples]

### Example 1. Preparation of compositions including garlic extract, black tea extract, and guava extract

A cosmetic composition was prepared by mixing each of the extracts prepared in Preparation Examples 1 to 3 in the same amount (33.3 wt%). Unless otherwise indicated in the present specification, an amount is expressed in wt%.

### Example 2. Preparation of composition including garlic extract, black tea extract, guava extract, guava leaf extract, luffa cylindrica fruit extract, and water lily flower extract

A cosmetic composition was prepared by mixing 1.5 wt% of each of the extracts prepared in Preparation Examples 1 to 3, 1.0 wt% of each of the extracts prepared in Preparation Examples 4 and 5, 3.0 wt% of the extract prepared in Preparation Example 6, 2.0 wt% of an adjuvant (e.g., 1,2-hexanediol), 30.0 wt% of butylene glycol, and 58.5 wt% of purified water.

### Example 3. Preparation of freeze-dried product of composition including garlic extract, black tea extract, guava extract, guava leaf extract, luffa cylindrica fruit extract, and water lily flower extract

A freeze-dried product of the composition prepared in Example 2 was prepared by freeze-drying. The freeze-dried product had a yield of about 500 mg/L.

### [Comparative Examples]

### Comparative Examples 1 to 3. Preparation of composition including extract

A garlic extract, a black tea extract, and a guava extract of Comparative Examples 1 to 3 were prepared in the same manner as in Preparation Examples 1 to 3.

### [Experimental Examples]

### Experimental Example 1. Confirmation of inhibitory effect on membrane fusion

The inhibitory effect of the composition according to an embodiment on the membrane fusion was confirmed. Also, since myricetin was predicted as an active ingredient, the inhibitory effect of 100 µM of myricetin (Sigma-Aldrich) on the membrane fusion was also confirmed.

First, a soluble nethylmaleimide-sensitive factor attachment protein (SNAP) receptor protein was overexpressed in transformed *E. coli,* and then the expressed protein was selectively purified and obtained by using glutathione agarose beads. Then, 1-palmitoyl-1,2-dioleoyl-sn-glycero-3-phosphatidylcholine (POPC, 62 mol%), 1,2-dioleoyl-sn-glycero-3-phosphatidylserine (DOPS , 35 mol%), 1,2-dioleoyl-sn-glycero-3-phosphoserine-N-(7-nitro-2-1,3-benzoxadiazol-4-yl) (NBD-PS , 1.5 mol%), and a fluorescent material, rhodamine (rhodamin-PE, 1.5 mol%) were mixed to prepare a 10 mM fluorescent substance-labeled liposome (v-vesicle). Meanwhile, DOPS and POPC were mixed to a molar concentration of 35:65 to prepare a 50 mM liposome (t-vesicle) that is not labeled with a fluorescent substance. Then, purified synaptosomal-associated protein, SNAP-25, and syntaxin 1a were mixed at a molar concentration of 1: 1 and allowed for a reaction for 1 hour at room temperature, followed by mixing with the liposome that is not labeled with a fluorescent substance at a molar ratio of 100:1. Also, vesicle-associated membrane protein 2 (VAMP-2) was mixed with the fluorescent substance-labeled liposome at a molar ratio of 50:1. These two types of the liposomes were dialyzed and mixed at a molar ratio of 1:9 (v-vesicle:t-vesicle), and treated with 100 *µ*g/mℓ of each of the extracts of Example 1 and Comparative Examples 1 to 3. Then, the fluorescence intensity thereof was measured.

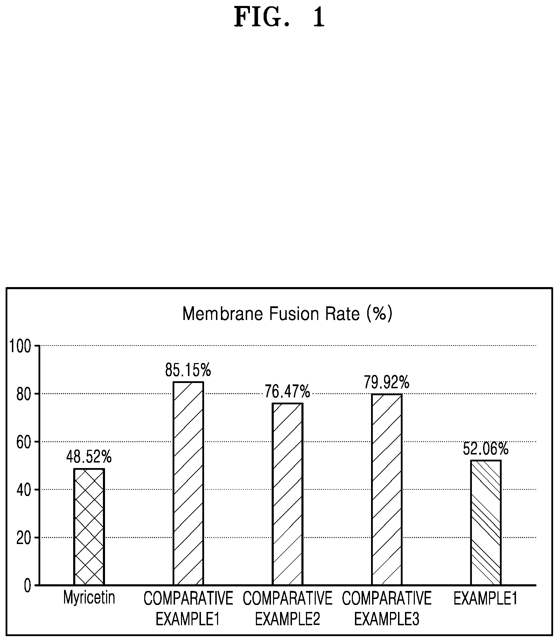

FIG. 1 shows a result of confirming the membrane fusion rate by the treatment with the composition and myricetin according to an embodiment.

As a result, as shown in FIG. 1, the composition of Example 1 including all of the garlic extract, the black tea extract, and the guava extract showed a significantly reduced membrane fusion rate compared to the extracts of Comparative Examples 1 to 3 each containing the extract of single component, and the membrane fusion rate was most significantly reduced by myricetin.

That is, the composition according to an embodiment inhibits the formation of SNARE complex by acting specifically on the SNARE protein, and thus may exhibit a synergistic activity on the inhibition of membrane fusion. Also, it was confirmed that the inhibition effect on membrane fusion may be due to myricetin.

Accordingly, the composition not only inhibits the secretion of sweat and/or sebum by blocking the release of neurotransmitters and inhibits the generation of wrinkles by musculation or sympathetic and parasympathetic nerves, but also ameliorates wrinkles already generated. Such effects may be exhibited also due to the myricetin.

### Experimental Example 2. Confirmation of myricetin detection

It was confirmed whether myricetin included in the composition according to an embodiment was detected.

In detail, the composition of Example 3 was dissolved in the same volume of methanol (MeOH) as the original composition, filtered through a 0.45-µg PVDF filter, and then quantified by using the reverse-phase HPLC system with a UV system and the INNO C18 column. Here, the column temperature was maintained at 35 °C, the flow rate was maintained at 1 mL/min, the sample input was set at 10 microliter, and the measurement wavelength was set at 350 nm. For a fluidized phase, a 0.5 % acetic acid aqueous solution (A) and acetonitrile (B) were used, and a concentration gradient was set as 80% A at 0 min, 70% A at 30 min, and 80% A at 35 min. By using a standard curve constructed using 1 ppm to 100 ppm of myricetin, the amount of myricetin included in the sample was calculated.

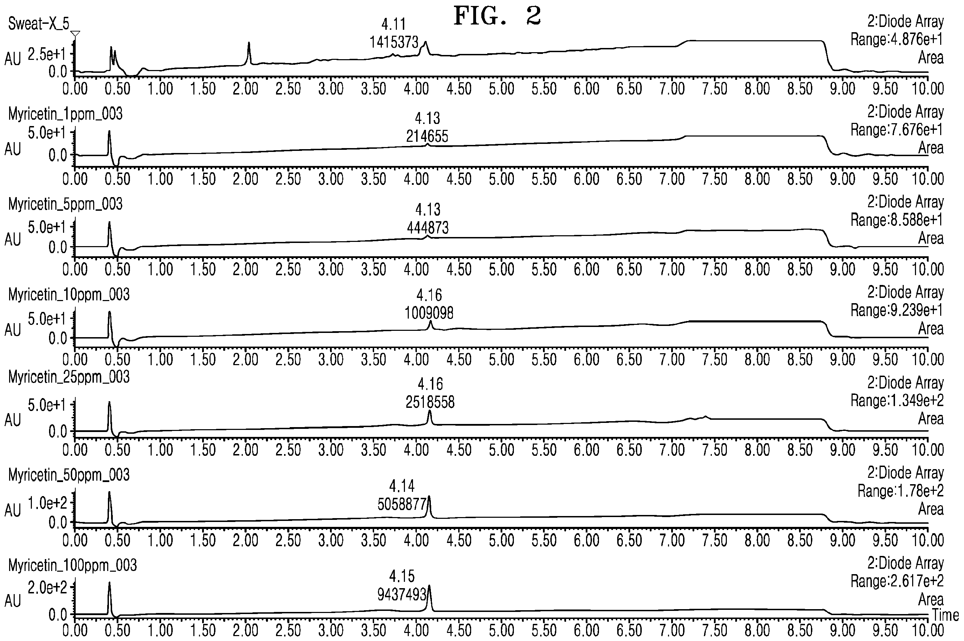

As a result, as shown in FIG. 2, the myricetin was detected in Example 3, and when calculated by using the standard curve, it was confirmed that the amount of myricetin in the composition of Example 3 was 13.85 mg/L.

Therefore, it was confirmed that the composition according to an embodiment contained the myricetin as an active ingredient.

### Experimental Example 3. Confirmation of effects on inhibition of SNARE protein and suppression of neurotransmitter release in human skin tissue

First, the inhibitory effect of the composition and myricetin according to an embodiment on the SNARE protein was confirmed. In detail, the human skin explant, which has been disinfected with povidone and completely underwent fat removal, was evenly cut with an 8-mm biopsy punch, and then cultured for 24 hours in a transwell to which 1.5 mL medium (DMEM + 10 % FBS + 10 µg / mL insulin + 10 ng / mL hydrocortisone + 2 mM L-glutamine) was added. 10 µL of each of the composition of Example 3 and the myricetin sample was treated evenly over the entire skin at the respective set concentrations, and then further cultured for 72 hours. As a positive control, botulinum toxin type A (botox cosmetics) was used. The cultured tissues have been completely cultured were fixed with formalin for fluorescence staining with VAMP2 (ab215721) which is one of the SNARE proteins. The fluorescence-stained tissues were photographed under a confocal microscope, and the fluorescence intensity of VAMP2 was analyzed by using Image J.

Next, the inhibitory effect of the composition and myricetin according to an embodiment on the release of neurotransmitters was confirmed. In detail, the human skin explant, which has been disinfected with povidone and completely underwent fat removal, was evenly cut with an 8-mm biopsy punch, and then cultured for 24 hours in a transwell to which 1.5 mL medium (DMEM + 10 % FBS + 10 µg / mL insulin + 10 ng / mL hydrocortisone + 2 mM L-glutamine) was added. 10 µL of each of the composition of Example 3 and the myricetin sample was treated every day for 3 days at the respective set concentrations. As a positive control, botulinum toxin type A (botox cosmetics) was used. The cultured tissues were stored in LN₂. After uniformly homogenizing the tissues stored in LN₂, proteins were quantified by using the Bradford assay. Each sample was adjusted to the same amount of the proteins, and the amount of a neurotransmitter, acetylcholine, was measured at Ex/Em 535/587 nm by using the choline/acetylcholine assay kit (ab65345).

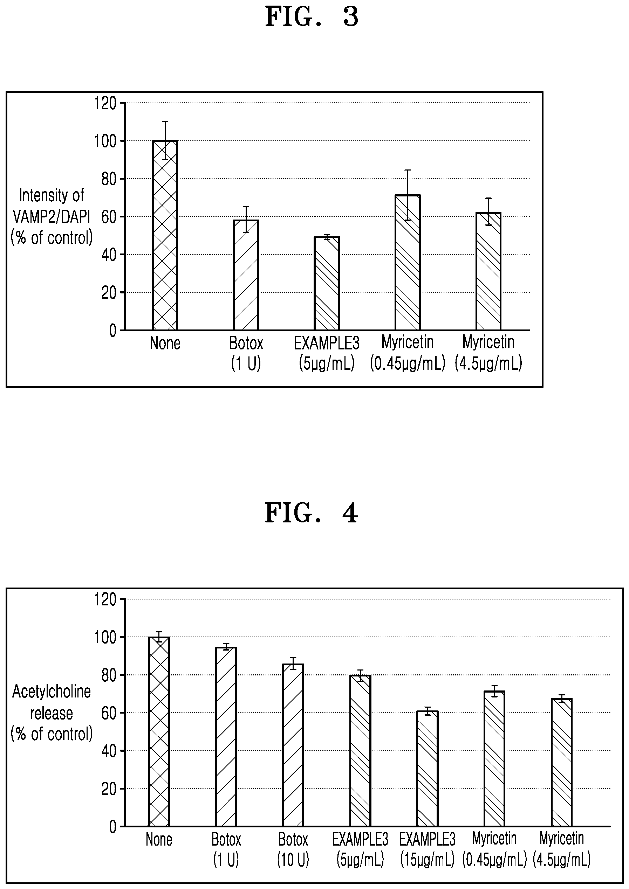

FIG. 3 shows a result of confirming the VAMP2 level by treatment with the composition or myricetin according to an embodiment.

FIG. 4 shows a result of confirming the acetylcholine level by treatment with the composition or myricetin according to an embodiment.

As a result, as shown in FIGS. 3 and 4, the VAMP2 level and the amount of acetylcholine released in the skin tissue were significantly reduced by the composition of Example 3 or the myricetin.

That is, the composition according to an embodiment inhibited the formation of SNARE complex by acting specifically on the SNARE protein and accordingly inhibited the membrane fusion so that the release of neurotransmitters was inhibited. It was confirmed that such effects may be exhibited by the myricetin.

Accordingly, the composition not only inhibited the secretion of sweat and/or sebum by blocking the release of neurotransmitters and inhibited the generation of wrinkles by musculation or sympathetic and parasympathetic nerves, but also ameliorated wrinkles already generated. It was confirmed that such effects may be exhibited by the myricetin.

### Experimental Example 4. Confirmation of inhibitory effect on sebum secretion

The inhibitory effect of the composition according to an embodiment on the sebum secretion was confirmed.

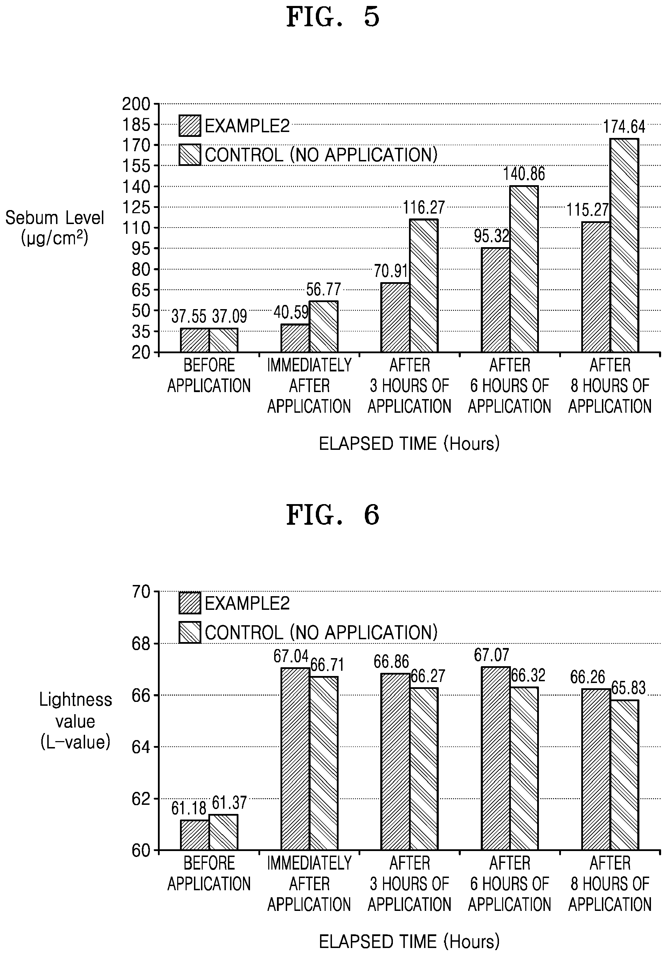

In detail, the composition of Example 2 was applied to one side of the face of 22 women in their 20s to 50s to be absorbed, and then a reference color sample (Dr. Jart+ The Makeup Fit Cushion 01 Light product) was applied thereto (excluding the forehead). Thereafter, after washing the face with an existing cleanser, the face was lightly patted with a paper towel to remove moisture, and then rested for 30 minutes under constant temperature and humidity conditions (20 °C to 24 °C, 40 % to 60 % RH). After the face of a test subject was photographed, sites for the measurement were divided, and the amount of sebum secreted on the skin was measured 5 times each by using a sebumeter before the application, immediately after the application, and 3 hours, 6 hours, and 8 hours after the application. The average value of the measured values is shown in FIG. 5.

FIG. 5 shows a result of confirming changes in the amount of skin sebum by the composition according to an embodiment for each time period.

As a result, as shown in FIG. 5, it was confirmed that the amount of sebum secreted was 56.77 µg/cm² immediately after the application, 116.27 µg/cm² after 3 hours of the application, 140.86 µg/cm² after 6 hours of the application, and 174.64 µg/cm² after 8 hours of the application, in the case where the composition of Example 2 was not applied. Meanwhile, in the case where the composition of Example 2 was applied, it was confirmed that the amount of sebum secreted was 40.59 µg/cm² immediately after the application, 70.91 µg/cm² after 3 hours of the application, 95.32 µg/cm² after 6 hours of the application, and 115.27 µg/cm² after 8 hours of the application, and accordingly that there was a statistically significant difference from immediately after the start of the test after 8 hours.

That is, when the composition of Example 2 was applied, it was confirmed that the amount of sebum secreted was low at a statistically significant level (p<0.05) until 8 hours of the application, compared to the case where the same composition was not applied.

Therefore, the composition according to an embodiment was able to decrease or inhibit the sebum secretion, which may be also due to myricetin as an active ingredient.

### Experimental Example 5. Confirmation of makeup durability effect

The effect of the composition according to an embodiment on the makeup durability was confirmed.

In detail, the composition of Example 2 was applied to one side of the face of 22 women in their 20s to 50s to be absorbed. After washing the face with an existing cleanser, the face was lightly patted with a paper towel to remove moisture, and then rested for 30 minutes under constant temperature and humidity conditions (20 °C to 24 °C, 40 % to 60 % RH). Next, a reference color sample (Dr. Jart+ The Makeup Fit Cushion 01 Light product) was applied to the testing site (except for the forehead), and the face of the test subject was photographed immediately after the application and after 3 hours, 6 hours, and 8 hours of the application. Here, the changed in the skin color (L-value) of the test subject was measured by using a chromameter.

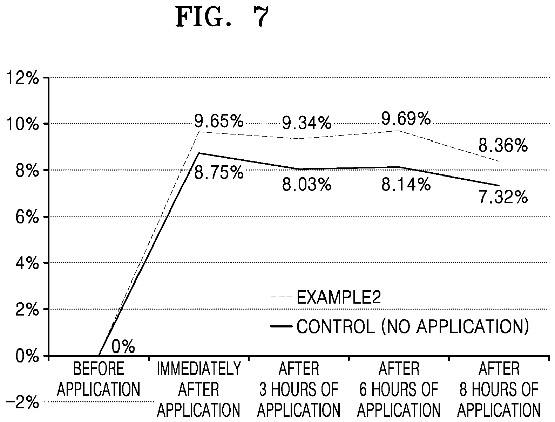

FIGS. 6 and 7 show a result of confirming changes and rates of skin color change by the composition according to an embodiment for each time period.

As a result, as shown in FIGS. 6 and 7, in both cases where the composition of Example 2 was applied or not applied, the measured values for the skin color before and after the application showed the same pattern. After the application of the standard color sample, it was confirmed that the skin color changed at a statistically significant level (p<0.05) by the makeup effect of the colored cosmetics. However, compared to the case where the composition of Example 2 was not applied, the skin color in the case where the same composition was applied increased at a high level, and it was confirmed that such an increase was statistically significant from 3 hours after application to 8 hours after application.

That is, the composition according to an embodiment not only exhibited an effect on the skin color improvement by color makeup, but also improved makeup durability, and such effects may be due to myricetin which is an active ingredient.

According to the one or more embodiments, a composition including a garlic extract, a black tea extract, and a guava extract, or a composition including myricetin may inhibit membrane fusion by the formation of SNARE complex and inhibit the release of neurotransmitters, so that the wrinkles may be ameliorated and the secretion of sweat and sebum may be inhibited, thereby exhibiting effects of makeup durability and preventing or treating neuromuscular-related diseases.

It should be understood that embodiments described herein should be considered in a descriptive sense only and not for purposes of limitation. Descriptions of features or aspects within each embodiment should typically be considered as available for other similar features or aspects in other embodiments. While one or more embodiments have been described with reference to the figures, it will be understood by those of ordinary skill in the art that various changes in form and details may be made therein without departing from the spirit and scope of the disclosure as defined by the following claims.

## Claims

1. A cosmetic composition for improving a skin condition, comprising, as an active ingredient, a garlic extract, a black tea extract, and a guava extract.

2. The cosmetic composition of claim 1, wherein the garlic extract, the black tea extract, and the guava extract are mixed at a weight ratio in a range of about 1: 0.1:0.1 to about 1:10:10.

3. The cosmetic composition of claim 1, wherein the garlic extract, the black tea extract, or the guava extract is obtained by performing an extraction process on garlic, black tea, or guava by using, as a solvent, water, a C1-C4 lower alcohol, or a mixture thereof.

4. The cosmetic composition of claim 1, wherein the skin condition is selected from skin elasticity amelioration, reduction in fine line appearance, wrinkle amelioration, eye-widening effect, lifting of the corners of the mouth, reduction in muscle mass, and flattening of line unfolding from the upper lip.

5. The cosmetic composition of claim 1, further comprising a guava leaf extract, a luffa cylindrica extract, and a water lily flower extract.

6. The cosmetic composition of claim 1, wherein the cosmetic composition is for suppressing secretion of sweat or sebum or for improving or enhancing makeup durability.

7. A pharmaceutical composition for preventing or treating neuromuscular-related diseases, comprising, as an active ingredient, a garlic extract, a black tea extract, and a guava extract.

8. The pharmaceutical composition of claim 7, wherein the neuromuscular-related disease is selected from muscle tension, muscle spasm, hemifacial spasm, adult onset spasmodic torticollis, anal fissure, blepharospasm, facial muscle spasm, cerebral palsy, headache, migraine, muscle pain, strabismus, temporomandibular joint disorder, nerve pain, overactive bladder, urgency urinary incontinence, rhinitis, paranasal sinusitis, acne, pore enlargement, dystonia, dystonic contraction, hyperhidrosis, vocal cord dysfunction, and myocardial damage.

9. A cosmetic composition for improving a skin condition, comprising myricetin as an active ingredient.

10. The cosmetic composition of claim 9, wherein myricetin is derived from: chemical synthesis; biosynthesis using a microorganism; or a plant or a mushroom selected from broccoli, cauliflower, cabbage, Chinese cabbage, kalian, green chili, red chili, bell pepper, bird chili, Chinese chives, onion, garlic, belimbi, yam, tapioca, sweet potato, papaya, cashew, fern, soybean, mungbean, okra, winged bean, French bean, pea, string bean, petai, peria, brinjal, angular loofa, snake gourd, pumpkin, sengkuang, guava, carrot, white radish, red spinach, bayam duri, kangkung, wolfberry, drumstick, celery, limau purut, daun turi, betel, pandan, lemon, semambu, kesom, Maman (*Gynandropsis gynandra),* Kadok, cekur manis, selom, pegaga, bunga kantan, plantain, asam gelugor, turmeric, mint, oyster mushroom, and black tea.

11. The cosmetic composition of claim 9, wherein the skin condition is selected from skin elasticity amelioration, reduction in fine line appearance, wrinkle amelioration, eye-widening effect, lifting of the corners of the mouth, reduction in muscle mass, and flattening of line unfolding from the upper lip.

12. The cosmetic composition of claim 9, wherein the cosmetic composition is for suppressing secretion of sweat or sebum or for improving or enhancing of makeup durability.

13. A pharmaceutical composition for preventing or treating neuromuscular-related diseases, comprising myricetin as an active ingredient.

14. The pharmaceutical composition of claim 13, wherein the neuromuscular-related disease is selected from muscle tension, muscle spasm, hemifacial spasm, adult onset spasmodic torticollis, anal fissure, blepharospasm, facial muscle spasm, cerebral palsy, headache, migraine, muscle pain, strabismus, temporomandibular joint disorder, nerve pain, overactive bladder, urgency urinary incontinence, rhinitis, paranasal sinusitis, acne, pore enlargement, dystonia, dystonic contraction, hyperhidrosis, vocal cord dysfunction, and myocardial damage.
